# EUROPEAN PATENT APPLICATION

(11) **EP 1 110 524 A1**
(43) Date of publication of application: **27.06.2001**
(21) Application number: 99125934.2
(22) Date of filing: 23.12.1999
(51) Int. Cl.: A61F 13/15, A61F 5/455

(54) **Liquid handling member with a membrane assembly comprising a membrane wetting region**

(71) Applicant: THE PROCTER & GAMBLE COMPANY, Cincinnati, Ohio 45202 (US)
(72) Inventor: Schmidt, Matthias, 65510 Idstein (DE); Ehrnsperger, Bruno Johannes, 65936 Frankfurt (DE); Poursanidis, Georgios, 60389 Frankfurt (DE)
(74) Representative: Kohol, Sonia

(57) **Abstract**

The present invention is a liquid handling member, which comprises a first zone and a second zone connected to a suction device, wherein the second zone circumscribes a second zone volume which is at least to 50% filled with a non-liquid fluid, and wherein the first zone and the second zone are separated by a membrane assembly which is capable of maintaining a pressure differential between the second zone and the first zone without permitting air to penetrate from said first zone to said second zone. The assembly comprises further a first porous membrane region oriented towards the first zone, and a second region oriented towards the second zone, which is capable of holding liquid, and which is in liquid communication with the pores of said porous membrane region.

## Description

### Field of the invention

The present invention relates to liquid handling members comprising a membrane material, such as can be useful is various liquid transporting members. In particular this can be applied in hygienic articles, such as external catheter, absorbent articles or the like.

### Specification/Description

Liquid transport systems exploiting membrane functionalities are known in the art.

US-A-5.678.564 describes a liquid removal system, wherein liquid removal is achieved by using an interface device, which is provided with a membrane. This membrane is capable of maintaining a vacuum on one side so that when liquid contacts the opposite side of the membrane the liquid passes through the membrane and is removed from the interface device by a maintained vacuum to a receptacle for disposal.

In order to be operational, the membrane must be wetted, both at the beginning of the liquid removal, as well as throughout the period of intended liquid removal, e.g. when several gushes of liquid are delivered. This can be achieved by prewetting the membrane with a liquid, such as glycerin, which is preferred as a prewetting agent because it will not readily dry out and will support the vacuum until the first wetting with the liquid which is intended to be removed, e.g. urine. Once the unit is wetted with urine, the glycerin is washed out, and the urine in conjunction with the membrane supports the vacuum.

However, whilst the problem of dry out before the first wetting has been identified and addressed by the glycerin addition, a problem remains when the system is intended for intermittent wettings with substantial periods between the wettings during which the urine can evaporate from the mebrane in the regions where the glycerin has been washed away.

If, as described in US-5.678.564, the inner region of the liquid removal system is vacuum, the evaporation can quickly result in drying out of the membrane, and the membrane will loose its functionality.

If, as described as an alternative embodiment in US -A- 5.678.564, the inner region is completely filled with the liquid, then the evaporation loss can be compensated by this liquid. However, in this instance this inner region needs to be filled before the first wetting, i.e. also during transportation.

Henceforth, there remains the need of providing a liquid removal system with an improved evaporation stability at lower initial weight of the overall system, and allowing a broader selection of liquids for the initial wetting.

Further, membranes loose their functionality generally as a function of the pore size of the largest pore. As soon as this is allowing gas or air to pass through, the membrane looses its functionality. In other terms, the bubble point pressure, i.e. the parameter describing the limitations of functionality of such membranes, is strongly depending on this maximum pore size. It would be highly desireable, to increase the functionality beyond this limitation.

Even further, once conventional membranes have lost their functionality after the largest pore is dried out, those cannot regain this functionality without re-activation of the system, which generally requires the intervention of a user. Henceforth, it would be desirable, to create sysstems, which have - at least to a certain degree - a "self repairing mechanism".

These limitations and problems of conventional liquid transport members comprising membranes have been overcome by the present invention.

Essentially, the approach is to provide membrane assemblies with a liquid reservoir which is in liquid communication with the pores of the membrane, even if the membrane assembly separates regions which can comprise predominantly a gas or vapour phase, i.e which are not necessarily completely filled with liquid.

### Summary:

The present invention is a liquid handling member, which has a first zone and a second zone connected to a suction device, wherein the second zone circumscribes a second zone volume which is at least to 50% filled with a non-liquid fluid, and wherein the first zone and the second zone are separated by a membrane assembly which is capable of maintaining a pressure differential between the second zone and the first zone without permitting air to penetrate from said first zone to said second zone. The assembly comprises further a first porous membrane region oriented towards the first zone, and a second region oriented towards the second zone, which is capable of holding liquid, and which is in liquid communication with the pores of said porous membrane region.

The second region of the membrane assembly can comprise a porous material with maximum pore size larger than the pore size of the membrane assembly, which can be an open porous material having a pore size of at least ten times the pore size of said first membrane material.

The second region of the membrane assembly can comprise a membrane material, which can be hermetically sealed with the first membrane, thereby circumscribing a membrane assembly inner region, which can be substantially filled with liquid. This assembly can further comprise liquid and gas impermeable wall regions. Either of the membranes, the seal, or the wall regions of this membrane assembly can be elastically deformable, thereby alowing the membrane assembly volume to change.

At least the pores of one of the membrane members of the membrane assembly can be filled with liquid prior to its intended use, which can be liquid having a lower vapor pressure than water under respetive temperatures.

Especially for the handling of aqueous systems, the first membrane has a maximum pore size of less than 50 µm, preferably of less than 30 µm, the second assembly membrane has a pore size of less than 200 µm or the open pore material has a pore size of less than 800 µm.

### Detailed description

A liquid removal system in the present context is considered to be a system, wherein a liquid is penetrating through a membrane by means of a potential difference, such as pressure differential like a suction or a vaccum. Whilst such a liquid can be of almost any type, the following description will describe the mechanisms by using aqueous liquids, such as salt solutions like urine. It will be apparent to the skilled person, to re-apply the teaching for other liquids, such as oily substances as have been discussed in more detail in PCT applications US 99/14644 or US 99/14645.

Such systems function by the principle that certain porous membranes under certain conditions can be permeable to liquids, but not to gases like air, as long as the "potential differential " such as the pressure differential between the two sides of such a membrane does not exceed a certain value characteristic for the material and the liquid within the pores of the material - the bubble point pressure. This latter is often expressed in "height of water column" which corresponds to the pressure exerted by such a column on the material under normal gravity conditions.

For aqueous liquids, the material for the membrane is preferably hydrophilic and has a pore size on the order of about 5 to about 30 µm, more preferably about 10 to 20 µm. Once the membrane has been wetted it will support a suction pressure typically corresponding to about 12.5 to about 150 cm column of water without permitting air to pass. Thus, if suction is applied to a first side of a wetted membrane, liquid contacting the membrane on the other side will be drawn by the suction through the membrane to the first side of the membrane, from where it can further be removed, for example by being sucked by means of a vaccum through a drain tube to a reservoir. As long as the filter material or membrane remains wet, air does not pass through the filter and suction is maintained without active pumping. If too much vacuum is applied to the membrane there is a risk that the bubble point of the membrane will be surpassed, thereby allowing air or gas to penetrate through, which can lead to a loss of the vacuum, and of the liquid handling functionality. Thus the amount of vacuum should approach as close as possible but not exceed the bubble point pressure.

In such a system, the membrane needs to be "hermetically sealed" to the other elements, which means that a gas (and especially air) can neither pass from the outside environment to the inside of the member, when the membrane is saturated with liquid, as long as the pressure differential does not exceed the bubble point pressure.

The loss of functionality can also occur when the membrane is dried out by other mechanisms, such as by evaporation to either side of the membrane, e.g. the moisture that saturates the membrane may evaporate into the vaccum, or it can evaporate to the ambient on the other side.

Henceforth, it is desired for the membrane to be saturated with liquid. This liquid not necessarily has to be the liquid which is intended to be handled during the intended use.

If the liquid handling member comprises an inner region which is completely filled with liquid, there will generally be sufficient flexibility in the system to compensate under otherwise steady conditions for the loss of liquid by the evaporation, and the membrane will remain continuously saturated. However, such systems require this liquid filling to be established at the time of manufacturing, and needs to be maintained until and throughout its intended use. This can increase the weight and volume of the system, and hence is undesireable.

If - as for members according to the present invention - this inner region is not completely filled with liquid, but rather with a gas or vapour phase, drying of the pores to either or both sides of the membrane cannot be compensated by the liquid as decribed before, and thus the pores can completely dry out, and the membrane can loose its functionality.

As desribed for example in US-A-5.678.564, the membrane material can be prewetted during manufacture by an otherwise suitable liquid which has a low vapor pressure and thus is less prone to evaporation drying. Glycerin has been proposed as a prewetting agent because it will not dry out and will support the vacuum until the first wetting with urine. The aqueous liquid passes though the glycerin wetted filter material with no difficulty and a suction is supported by the glycerin. However, once the unit is wetted with the aqueous liquid, the glycerin is washed out, and the aqueous liquid supports membrane functionality as described before. Henceforth, if there is a further waiting time between this first loading and a further gush, risk of pores drying out and loss of membrane functionality is as before.

Henceforth, the present invention provides a structure, the membrane assembly, which permanently can maintain the pores of the membrane filled with liquid by providing a membrane wetting region, which is arranged in liquid communication with the pores of the membrane.

This membrane assembly must comprise a reservoir which can hold sufficient liquid to compensate for the losses from the pores. Thus, the reservoir should have a certain volume filled with liquid, which either can be the same as the liquid which is to be transported, or it can be a different one, such as one having a lower propensity to evaporate. The reservoir must further be able to release the liquid to the pores, thus have no constraining or rigid walls so as to reduce the respective reservoir volume. The reservoir can be completely circumscribed by a liquid impermeable wall, or can have a liquid permeable wall, or no wall material as such, and then retain the liquid therin by other mechanisms such as capillary suction mechanisms.

This reservoir must be in liquid communication with the pores of the membrane. This liquid communication can be achieved by the reservoir covering essentially the membrane surface. Alternatively, the major part and volume of the reservoir can be located somewhat remote from the membrane and have a connection to the membrane.

The term liquid communication refers to the mechanism, that the liquid is transferred from the reservoir to the membrane, i.e. there must be liquid flow path from the reservoir to the pores. This liquid flow can be achieved by capillary action or gravity driven flow or other pressure differential. The liquid flow path should not comprise capillaries or other elements, which retain the liquid more strongly than the pores.

The liquid communication needs to connect essentially all pores of the membrane, and most importantly for the larger pores.

Whilst there are various ways to realize the wetting functionality of the membrane assembly, the following describes particular embodiments, which should not be considered limiting in any way.

In a first approach, the present invention provides a further membrane that is added to the assembly to work in cooperation with the first membrane. Actually both membranes together can be seen to function as one conventional membrane as discussed in the above.

The first of these membranes has exactly the same functionality, requirements, as well as properties and parameter as the single one in the above arrangements. The second one can have a larger pore size, and pores sizes of up to about ten times the pore size of the first membrane have been found to function satisfactorily.

The two membranes need to be hermetically sealed, i.e. they must circumscribe a volume which is separated from the outside. This sealing can be achieved by various techniques, such as by using adhesives, or heat sealing. Optionally, the sealing can comprise other wall materials, such as film materials or the like, which are essentially liquid and gas tight, at least up to the point where the respective membrane materials loose their functionality.

The two membranes should define an region to accommodate an appropriate amount of liquid. This inner region between the two membranes can be - apart form the liquid which is filled therein to maintain both membranes wetted - empty, or there can be a filler material, such an an open resilient material, or other spacer, such as springs, elastic corrugations, and so on.

Preferably, the second membrane has a bubble point pressure of less than the bubble point pressure of the first membrane, but it should have a bubble point pressure of more than 0.5 cm height of water column, preferably more than 2.0cm height of water column. The two membranes can have the same surface area.

In order to provide the increased evaporation resistance, liquid can be filled into the volume between the two membranes, for example during manufacture of the membrane assembly. Alternatively, the assembly can be filled by the user prior to the application of the assembly, or it can be filled by the first liquid contacting the member. When liquid evaporates from the pores, and this liquid is refilled from the reservoir region, this must have a mechanism to compensate for the volume change as created by this refilling mechnism, such as by certain elasticity, or by having a compensation volume.

The membranes can be any membrane suitable for a single membrane port region such as described in US-A-5.678.564 or PCT application US 98/13497. Exemplary materials are nylon meshes such as commercially available from Sefar, Rüschlikon, Switzerland, e.g. type 03-10/2.

The membrane materials may be of homogeneous composition, such as the previously described woven meshes. The membrane materials can also be composites, such as by comprising a support layer and the like. Also, the dual membranes can form a composite material, which can include for example spacer materials to create the volume for receiving the liquid. Such composites can use flat structures, or other three-dimesionally shaped ones, such as decscribed in co-pending PCT application "Liquid handling members comprising three-dimensionally shape membranes", attorney docket reference CM-2258FQ.

The membrane can also be made of inhomogeneous materials, such as by being foamed structure with larger pores in the inner part, which can provide a higher permeability, or a higher internal volume, and with smaller pores towards the surface, so as to create the appropriate bubble point pressure.

In a second approach, the reservoir region can also be realized by combining a first membrane as described in the above with a region having essentially no membrane functionality, respectively having a too small bubble point pressure to have such a functionality.

Such a reservoir should have a sufficiently open structure so as to not limit flow properties and to provide sufficient volume for the liquid to be hold therein.

It is not required, that the liquid is strongly hold in this reservoir, however the capillary pressure in this reservoir should not be less than corresponding to the thickness of the reservoir, i.e its maximum distance from the membrane region. The thicker this region is, i.e. the farther away the pores are arranged from the membrane surface, the smaller the pores should be . For relatively thin structures, the pores can have sizes of up to 800 µm and still largely prevent evaporation of a membranes having pores of about 20 µm.

Such materials can be exemplified by open nylon nets having struts of approximately 0.8 mm thickness, such as available from Villforth Siebtechnik GmbH, Reutlingen, Germany.

The combination of either of the two approaches for creating a reservoir region, or their combination can be comprised in an overall membrane assembly, such as decribed in PCT application 98/13497 and could therein replace "port" membranes, such that the overall liquid handling member then can comprise a first port region, for example a liquid inlet port region to receive the liquid to be transported, made of the membrane assembly according to the present invention, a second port region, for example a liquid outlet port region, which may also be a membrane assembly, or which can also be a single membrane. An inner region would then be defined between the inlet and the outlet region, i.e. between the innermost of the two dual membranes, and the respective other port.

Instead of having an inlet and an outlet port region, there also could be an inner region capable of receiving and storing the liquis, such as by being made of expandable material.

The overall system can further comprise means to create a liquid suction force, such as by creating a vacuum, using pumps, or other suction creating elements like absorbent materials, or osmotic materials. Such an suction creating element can also be an ultimate storage receptacle, which can be connected to the membrane by a drain tube or by any other liquid connection means.

Useful systems are described in more detail in US-A-5.678.564 or PCT application 98/13479.

The membranes may separate two disconnected regions, such as an entrance zone on the liquid receiving side, and a second zone connected to the vacuum or suction source, with corresponding hermetical sealing and separation.

The membranes and membrane assemblies according to the present invention can be used in a variety of applications, which can impose other requirements to the overall design. A more detailed discussion can be found in CM 1841. For example in personal hygiene applications, comfort for the wearer is important, and thus softness, skin compatibiltiy, shape of the total article and the like are important.

Structures according to the present invention exhibit various effects and benfits.

First, they provide an improved mechanism to overcome the effects of evaporation from the pores. If this evaporation would result in drying out pores, the membrane and the liquid handling member would loose its functionality.

When applying the present invention, the pores maintain the liquid filling. This is also maintained for subsequent liquid loadings, such as can readily occur in hygiene articles.

A test method to assess this effect is the membrane stability test

For this test, a membrane material or a membrane assembly is covering a closed suction chamber, which is connected to a suction creating device, such as a laboratory Pelaeus ball. It should be able to create a suction of approximately the bubble point pressure of the membrane. Further, this suction chamber is connected to a tube, which leads into a liquid reservoir filled with a suitable test liquid, such as destilled water, synthetic urine, or 0.9 % saline solution which can be open to the atmosphere. The reservoir is suitably placed under the suction chamber, with a height differential of about the height corresponding to the bubble point pressure of the membrane.

The complete set up is placed in a constant temperature/constant humidity chamber, such as 25 °C/ 50% RH and allowed to equilibrate.

Upon start of the experiment, the membrane is activated such as by being wetted with a liquid, also equilibrated at 25°C. A portion of the liquid can be drawn into the suction chamber, as long as there is sufficient gas in the chamber to prevent free liquid from wetting the membrane or membrane assembly.

The suction device is now activated to create an underpressure in the suction chamber, which will suck liquid residing on the membrane therethrough, and which also will suck liquid from the reservoir up into the tube connecting the reservoir with the suction chamber. This liquid column is used to adjust the suction as created by the suction device to 80% of the bubble point pressure of the membrane.

The system is now left to equilibrate and to assess the stability to drying over the desired time period, while the height of the liquid in the connection tube is monitored as a function of time at suitable intervals.

After the inital equilibration, this height will remain constant, as the suction is not sufficient to allow air to penetrate through the membrane. If the height drops during the intial period of approximately 10 minutes, the system needs to be re-established or reactivated.

Once it has been established, that the system is stable, a second liquid load can be applied to the membrane, which will be readily sucked therethrough, or - provided the tubing is sufficiently wide - remain within the suction chamber, which is not detrimental as long as it is not wetting the membrane or membrane assembly.

Now, the liquid column height in the tubing is re-adjsuted to the value as before the second loading, and the stability of the system can be re-evaluated as described before. If desired, the loading and stability waiting cycle can be repeated.

This method will enable to compare in particular the following systems:

A pure membrane material, which will loose its functionality upon drying out of the first pore. This functionality can be re-established upon re-activation, i.e. wetting the pores (such as by a second loading) upon re-establishemnt of a suction pressure at that time.

A glycerine treated membrane material, which will be stable for a significantly longer period as compared to the pure material. However, after application of a second loading, this will remove the glycerine from the pores, and thus leave the pores prone to drying as they were for the untreated material.

A dual membrane system as described hereinbefore will maintain its stability over extended periods over multiple repeated gushes.

An membrane assembly having a membrane and a nylon mask will maintain its stability essentially identical to the dual membrane system - also for repeated loadings - as long as the pore size to layer thickness ratio is not too excessive, upon which it would reach the functionality of the untreated - or in case of repeated loadings - of the glycerine treated material.

In addition to the stability improvements as desribed above it has surprisingly been found, that a dual membrane assembly as well as an open pore region assembly exhibit higher bubble point pressures than the membrane material used therin alone.

This effect can suitably be assessed by using the test equipment as described for the stability test, and increasing the suction in the suction chamber to the bubble point pressure, instead of testing the stability.

Yet a further beneficial effect of the present invention relates a certain self repairing mechanism for membrane assemblies, for which one or more pores were dried out, such as by an exceeding high suction - provided, this would not cause the suction to collapse. In such cases, the liquid of the reservoir region can readily fill up the dry pore, and thus re-eastblish the membrane functionality.

## Claims

1. A liquid handling member , comprising a first zone, a second zone connected to a suction device,
wherein said second zone circumscribes a second zone volume which is at least to 50% filled with a non-liquid fluid,
said first zone and said second zone being separated by a membrane assembly
which is capable of maintaining a pressure differential between the second zone and the first zone without permitting air to penetrate from said first zone to said second zone,
characterized in that said membrane assembly comprises a first porous membrane region oriented towards the first zone,
and a second region oriented towards the second zone, which is capable of holding liquid, and which is in liquid communication to the pores of said porous membrane region.

2. A Liquid handling member acording to claim 1, wherein said second region of the membrane assembly comprises a porous material with maximum pore size larger than the pore size of the first porous membrane region.

3. A Liquid handling member according to claim1 or 2 wherein said second region is an open porous material.

4. A Liquid handling member according to claim 3, wherein the pore size of said open porous material is more than ten times the pore size of said first membrane material.

5. A Liquid handling member according to any of claims 3 or 4 wherein said second porous material is a membrane material.

6. A Liquid handling member acording to claim 5, wherein said second porous material and said first porous membrane are hermetically sealed, thereby circumscribing a membrane assembly inner region.

7. A Liquid handling member according to claim 6, wherein said membrane assembly inner region is substantially filled with liquid.

8. A Liquid handling member according to any of claims 1 to 7 wherein said membrane assembly further comprises a liquid and gas impermeable wall region.

9. A Liquid handling member according to any of claims 1 to 8, wherein at least one of said membrane assembly membranes or said membrane assembly wall region is elastically deformable.

10. A Liquid handling member according to any of the preceding claims, wherein at least the pores of at least one of the membrane assembly membrane regions is filled with liquid prior to its intended use period.

11. A Liquid handling member according to claim 10, wherein said liquid is water disolvable, and has a vapour pressure at room temperature of less than the one of water at the respective temperature.

12. A Liquid handling member according to any of the proceeding claims wherein said second membrane assembly membrane has a maximum pore size larger than the maximum pore size of said first membrane assembly membrane.

13. A Liquid handling member according to claim 12, wherein said first membrane assembly membrane has a maximum pore size of less than 50 µm, preferably less than 30 µm.

14. A Liquid handling member according to claim 13, wherein said second membrane assembly membrane has a maximum pore size of more than 50 µm, preferably of more than 100 µm.

15. A Liquid handling member according to claim 14, wherein said second membrane assembly membrane has a maximum pore size of less than 200 µm.
